# EUROPEAN PATENT APPLICATION

(11) **EP 1 260 918 A2**
(43) Date of publication of application: **27.11.2002**
(21) Application number: 02011256.1
(22) Date of filing: 22.05.2002
(51) Int. Cl.: G06F 17/30

(54) **Database model, tools and methods for organizing information across external information objects**

(30) Priority: 22.05.2001 US 863115
(71) Applicant: Agilent Technologies Inc, Palo Alto, CA 94306-2024 (US)
(72) Inventor: KUCHINSKY, Allan, San Francisco, CA 94127 (US); MOH, David, San Francisco, CA 94131 (US); GRAHAM, Katherine D., San Jose, CA 95120 (US); CREECH, Michael L., Los Altos, CA 94024 (US)
(74) Representative: Schoppe, Fritz, Dipl.-Ing.

(57) **Abstract**

A system for organizing information across external information objects, comprises a results manager (20) for importing and viewing detailed experimental results as one type of representation of external information objects; a collection manager (30) for creating and manipulating collections of items representing external information objects; a story editor (40) for providing a narrative structure for textually organizing information about interactions between items, collections or items and collections; and a diagram editor (50) for incorporating items, collections or items and collections, into a graphical representation of a story; wherein an update of information contained in any one of the components comprising the results manager, collection manager, story editor, and diagram editor is made in the remainder of the components. Experimental data is verified and validated by overlaying items selected from the results manager onto a textual story provided in the story editor (40) or onto the graphical story provided in the diagram editor (50). The overlaid items are compared with the information in the textual story or the graphical story, and the result of the comparison is indicated in the story editor or in the diagram editor.

## Description

### CROSS-REFERENCE

This application is a continuation-in-part application of Application Serial No. 09/863,115, filed May 22, 2001, and titled "Software System for Biological Storytelling", which is incorporated herein by reference in its entirety and to which application we claim priority under 35 USC §120.

### FIELD OF THE INVENTION

The present invention pertains to software systems supporting the information synthesis activities of organizing, using, and sharing diverse, complex information.

### BACKGROUND OF THE INVENTION

As in many fields, research in molecular biology moves through an initial phase involving the formulation of models or hypotheses, into a middle phase where these hypotheses are tested through experiment.

In the early phase of model building and hypothesis formation, the user engages in speculation and hypothesis formation, identifying key elements, genes and proteins in molecular biology, and possible interactions of those key elements. In this early phase, the user is inferring causal relationships from correlations in test data, forming hypotheses which are to be refined and possibly tested.

The user in the field of molecular biology faces a daunting task in this early phase of model building. Unlike earlier endeavors where the number of possible variables was small, and experiments few and contained, users in molecular biology deal with enormous problems of scope.

Key elements, such as genes or proteins of interest, may number in the thousands, and the potential interactions may number in the billions. A single microarray experiment may produce megabytes of numerical data. The data is too large in scope to be held in the user's head.

To add to this problem, the user is faced with piecing together information from diverse sources and in different forms. This information is also geographically diverse, both in content and form, and may include public and private databases, textual information from publications, and experimental data both raw and refined. This data is also at multiple levels of abstraction, ranging from raw numerical gene expression data from microarray experiments, to textual descriptions of cellular processes.

The user must synthesize information in various forms from various sources into high level models, when developing hypotheses and explanations. Often, there is. a need to consider multiple hypotheses and alternative explanations in parallel. Moreover, users often work in teams, so there is a need to accommodate multiple perspectives and different views of the same data. Further, hypothesis formulation is a "top-down" reasoning process, where as the exploratory analysis of detailed experimental data is a "bottom-up" process. In order to be effective in formulating hypotheses, the user needs to reconcile the "top-down" and "bottom-up" perspectives, to ensure that the "top-down" explanations are consistent with the actual experimental data.

Very few tools exist to support this abstraction and exploration process. What is needed is a system for assisting users in the organization, using, and sharing of this diverse biological information.

### SUMMARY OF THE INVENTION

The present invention provides a system for organizing information across external information objects which may include any and all of the following components: a results manager for viewing detailed experimental results; a story editor for providing a narrative structure for textually organizing information about interactions between items; a collection manager for creating and manipulating collections of items representing external information objects; a diagram editor for incorporating items, collections and interactions into a graphical representation of a story; and an object editor for adding or manipulating annotations to information within the system.

Means for importing experimental data from external sources may be provided with the results manager. For biological applications, these external sources include, but are not limited to DNA microarray experimental results, relative protein abundance measures derived from mass spectrometry and protein fragment data derived from gel electrophoresis experiments.

Multiple results manager viewers may be used simultaneously, for viewing and manipulating multiple sets of data.

The story editor component may also include means for importing information from external sources, in addition to the capability of allowing direct input thereto by the user. The story editor may be further provide with means for importing items from the other components. Each of the components may be provided with the capability of importing from the other components. The components may be linked so that editing information within one component automatically updates the other components in the same way.

The object editor is adapted to annotate an item or interaction with a textual description. Other components, such as the story editor, may also include means for annotating an item or interaction with a textual description.

The collection manager is adapted to group related items together as a collection. Further, collections may be nested, i.e., a collection may contain one or more other collections, in addition to single items. The collections may be free-form sets of items. The collection manager may be provided with means for text-mining scientific literature to form collections. The collection manager may be adapted to semi-automatically import information and form collections. The collections may include links to external information.

The system may further include means for overlaying information from one or more components onto another component.

The diagram editor may include means for generating nodes corresponding to items and means for generating links between nodes which correspond to interactions. The diagram editor may include means for adding arbitrary nodes or links to the graphical organization.

The system may further be provided with means for tagging each annotation made with the name of a user who created it and with a time stamp indicating the time of creation of that annotation. The annotations may include text, data, pointers to external objects and/or pointers to external data, for example.

The system may further include means for generating a web repository, wherein the web repository includes a web page for each item.

The system may further be provided with means for saving work in progress.

The story editor may include a syntax-directed tree editor having means for identifying players to describe entities that play an active role in a story described, and means for defining hypotheses about interactions between the players.

Further, the story editor may include means for summarizing the story described as a theme, means for defining alternative hypotheses describing possible alternative interactions between the players; and/or means for documenting supporting and opposing statements and/or citations in support of or in opposition to one or more hypotheses, respectively.

The story editor may be provided with means for importing items from scientific text, graphical data or experimental data.

A method of organizing information across external information objects is described to include: importing information of diverse types from diverse sources; organizing the information into concepts and categories using a free-form database model; and formulating and documenting tentative explanations and hypotheses using the free-form database model.

Further, the method may include the step of attaching citations to the information by cutting and pasting or dragging and dropping the citations. The citations may be selected from Web references, files, free-form text, and graphic elements, for example.

A web repository of the organized information, explanations and hypotheses may be provided, for access by others. The method may further include incorporating verification and feedback from others who access the organized information, explanations and hypotheses and provide verification and feedback.

Preferably, the systems and methods provided are for use in organizing biological information, but they are not limited thereto, and can be used for other informational organization applications.

A free-form database model, embodied in software components, is provided, to include: items which represent external information objects; collections of items; textual stories describing the items, collections and interactions between the items, collections, and items and collections; and graphical stories describing the items, collections and interactions between the items, collections, and items and collections.

The free-form database model may further be provided with means for saving and restoring work in progress.

A method of verifying and validating experimental data is provided to include: importing the experimental data into a results manager; overlaying the values of items-selected from the results manager onto a textual story provided in a story editor or onto a graphical story in a diagram editor; and comparing the overlaid items with the information in the textual story or graphical story.

The overlaying may be performed by selecting the cell in the results manager that corresponds to an experimental result for that item, for example. Both the diagram editor and the story editor have code that "listens" for column-selected events, which are fired when a cell in the table is selected. That "listener" code then calls the routines that do the overlaying automatically.

A computer-readable medium carrying one or more sequences of instructions from a user of a computer system user for organizing information across external information objects is provided, wherein the execution of the one or more sequences of instructions by one or more processors cause the one or more processors to perform the steps of: importing information of diverse types from diverse sources; organizing the information into concepts and categories using a free-form database model; and formulating and documenting tentative explanations and hypotheses using the free-form database model.

The formulation and documentation may include generating a story utilizing a story grammar and/or generating a graphical story.

A further step of attaching citations to the information by cutting and pasting or dragging and dropping the citations may be performed.

Still further, a web repository of the organized information, explanations and hypotheses may be provided for access by others. The step of incorporating verification and feedback from others who access the organized information, explanations and hypotheses and provide said verification and feedback may also be performed.

The information is preferably, but not necessarily, biological information.

A computer-readable medium carrying one or more sequences of instructions from a user of a computer system user for organizing information across external information objects is provided, wherein the execution of the one or more sequences of instructions by one or more processors cause the one or more processors to perform the steps of: generating a results manager for importing and viewing detailed experimental results as one type of representation of external information objects; generating a collection manager for creating and manipulating collections of items representing external information objects; generating a story editor based on a narrative grammar for incorporating said items and collections into the narrative grammar to form a story; generating a diagram editor for incorporating items, collections and interactions into a graphical representation of a story; and generating an object editor for adding or manipulating annotations to information within the system.

These and other objects, advantages, and features of the invention will become apparent to those persons skilled in the art upon reading the details of the systems, methods and tools as more fully described below.

### BRIEF DESCRIPTION OF THE DRAWINGS

The present invention is described with respect to particular exemplary embodiments thereof and reference is made to the drawings in which:

Fig. 1 shows examples of main windows of the present invention;

Fig. 2 shows an Object Editor for an item according to the present invention;

Fig. 3 shows a File menu according to the present invention,

Fig. 4 shows a Results Manager window according to the present invention;

Fig. 5 shows a Collection Manager window according to the present invention;

Fig. 6 shows a Collection Manager menu according to the present invention;

Fig. 7 shows a Web browser view of a story according to the present invention;

Fig. 8 shows a story in tree form, in a Story Editor according to the present invention;

Fig. 9 shows a story grammar according to the present invention;

Fig. 10 shows a generated Web page for an item according to the present invention;

Fig. 11 shows a Diagram Editor window according to the present invention; and

Fig. 12 shows a Tools menu according to the present invention.

### DETAILED DESCRIPTION OF PREFERRED EMBODIMENTS

Before the present system, tools and methods are described, it is to be understood that this invention is not limited to particular viewers, tools, commands or steps described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a viewer" includes a plurality of such viewers and reference to "the data set" includes reference to one or more data sets and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

The term "activation" refers to enhancement of the effects of a biological agent or stimulation of a biological or chemical process, for example.

The term "alternative" when used in the context of describing a biological story, refers to one choice among a number of possible explanations (or hypotheses) for a biological phenomenon.

The term "amino acid" refers to a molecular sub-unit of a protein, containing an amino group, carboxyl group, and side chain attached to a carbon atom.

The term "analysis" is used herein to refer to a separation of a material or abstract entity into its constituent elements, as a method of studying its nature or determining its essential features.

The term "annotation" is used herein to refer to an explanatory or critical note that may be associated with any item, collection, story element, diagram node, or diagram interaction.

The term "biological story" defines a high-level description or explanation of a complex biological process, formulated by a researcher, for example, the "story" of how a mutation in a gene may lead to a cascade of events leading to a form of cancer.

The term "bottom-up analysis" refers to an inductive process of inferring patterns, concepts, and other higher-level information, beginning from detailed, constituent data.

The term "canvas" is used to describe a user interface component, typically in a graphical or textual editor, upon which a user can enter information, such as sketches or notes.

The term "cell", when used in the context describing a data table, refers to the data value at the intersection of a row and column in a spreadsheet-like data structure; typically a property/value pair for an entity in the spreadsheet, e.g. the expression level for a gene.

The term "cell cycle" refers to the biological process and phases of division and proliferation of a living cell.

The term "cell localization" refers to the location in a cell where a given biological entity, such as a protein, is concentrated, e.g. the plasma membrane, cytosol, nucleus, or organelles.

A "citation" is a quotation from or reference to an authority.

The term "collection" refers to free-form groupings or sets of related information. Collections can also be called or thought of as "categories" or "concepts".

The term "Collection Manager" defines a software component and user interface for viewing and manipulating collections.

"Color coding" refers to a software technique which maps a numerical or categorical value to a color value, for example representing high levels of gene expression as a reddish color and low levels of gene expression as greenish colors, with varying shade/intensities of these colors representing varying degrees of expression.

"Copying/cutting and pasting" refers to a user interface technique for moving or copying a data item from one view to another. A typical mechanism for copying and pasting is to (1) select the data item to be cut/copied, (2) perform cut/copy operation, either via a menu or via keyboard sequence, such as Cntl-X, (3) select data item into which the moved/copied data item is to be incorporated, and (4) perform paste operation, either via a menu or via keyboard sequence, such as Cntl-V.

The term "data mining" refers to a computational process of extracting higher-level knowledge from patterns of data in a database. Data mining is also sometimes referred to as "knowledge discovery".

The term "Diagram Editor" refers to a software component for presenting and manipulating biological process diagrams, such as signal transduction pathways and protein/protein interaction maps. A Diagram Editor can be thought of as a graphical mechanism for putting together a biological story. More generally, a Diagram Editor can be used to present and manipulate process diagrams outside of the biological realm.

The term "diagram interaction" refers to the representation, in the Diagram Editor, of a process or relationship involving two or more biological entities in the case of a biological diagram, e.g. a protein/protein binding interaction or a protein/gene inhibitory interaction. More generally, diagram interaction refers to the representation of the process or relationship between two or more entities in a diagram by the Diagram Editor.

A "diagram node" or "node", is the representation, in the Diagram Editor, of a specific item, collection, or Player.

The term "dragging and dropping" refers to a user interface technique for moving or copying a data item from one view to another. A typical mechanism for dragging and dropping is to (1) select the data item to be cut/copied or moved, (2) while holding down the mouse button, move the mouse sprite over to the data item into which the moved/copied data item is to be incorporated, and (3) release the mouse button when mouse sprite is over the data item into which the moved/copied data item is to be incorporated. Holding down the Cntl- key when mouse button is depressed results in copying of the source item; otherwise, the source item is moved out of source position and into destination.

A "drop point" is a location where the mouse button is released during a drag/drop operation.

A "file chooser" is a user interface component for navigating a directory/folder tree and selecting a file desired for an operation, which is based upon file navigation mechanisms in Microsoft Windows and Apple Macintosh operating systems.

A "file header" is an auxiliary information pre-pended to a data file, typically used to define fields, value types, and other structural information about the data in the file; for example, specifying whether data in a particular column is to be treated as text or as a numerical value.

A "file menu" is a user-interface mechanism for choosing one of a number of possible file-related operations, e.g. importing a gene expression data set.

A "free-form data model" refers to a model for data representation and storage which, in contrast to a formal, fixed database model, allows for the entry of arbitrary data before the definition of database tables. This allows the user to "add data now, categorize later".

The term "differentiation" refers to a process by which unspecialized cells acquire specialized structural and functional properties.

The term "down-regulation" is used in the context of gene expression, and refers to a decrease in the amount of messenger RNA (mRNA) formed by expression of a gene, with respect to a control.

The term "Explanations" is used to refer to a set of assumptions, clarifications, and hypotheses that constitute the "plot" of a biological story.

"Gel electrophoresis" refers to a biological technique for separating and measuring amounts of protein fragments in a sample. Migration of a protein fragment across a gel is proportional to its mass and charge. Different fragments of proteins, prepared with stains, will accumulate on different segments of the gel. Relative abundance of the protein fragment is proportional to the intensity of the stain at its location on the gel.

The term "gene" refers to a unit of hereditary information, which is a portion of DNA containing information required to determine a protein's amino acid sequence.

"Gene expression" refers to the level to which a gene is transcribed to form messenger RNA molecules, prior to protein synthesis.

"Gene expression ratio" is a relative measurement of gene expression, wherein the expression level of a test sample is compared to the expression level of a reference sample.

A "gene product" is a biological entity that can be formed from a gene, e.g. a messenger RNA or a protein.

A "growth factor" refers to one of a group of peptides that is highly effective in stimulating cell division and/or differentiation of certain cell types.

A "heat map" is a visual representation of a tabular data structure of gene expression values, wherein color codings are used for displaying numerical values. The numerical value for each cell in the data table is encoded into a color for the cell. Color encodings run on a continuum from one color through another, e.g. green to red or yellow to blue for gene expression values. The resultant color matrix of all rows and columns in the data set forms the color map, often referred to as a "heat map" by way of analogy to modeling of thermodynamic data.

"HTML" or "HyperText Markup Language" refers to a system of standards used to tag the elements of a hypertext document; and is a standard for documents on the World Wide Web.

A "hypothesis" refers to a provisional theory or assumption set forth to explain some class of phenomenon.

"Hypertext" refers to data, as text, graphics, video, or sound, stored in a computerized document so that a user can move non-sequentially through a link from one document to another.

The term "Import Bio Data" refers to a user interface operation for bringing detailed experimental data into the software system.

An "Index Web page" is a Web page that consists of links to other Web pages, e.g. the index of all Collections in the current model.

The term "inhibit" refers to a decrease in the effects of a biological agent or a biological process.

The term "interaction" refers to a process or relationship involving two or more entities, e.g., biological entities such as a protein/protein binding interaction or a protein/gene inhibitory interaction.

The term "Issue Based Information System" refers to a class of computer software systems that provide an explicit data representation, usually in diagrammatic form, of the issues, positions, and arguments generated during a group deliberation. An issue based information system helps workgroups to document their lines of reasoning in coming to decisions on complex problems.

An "item" refers to a data structure that represents a biological entity or other entity. An item is the basic "atomic" unit of information in the software system.

The term "kinase" refers to an enzyme involved in signal transduction, typically by transferring a phosphate to another molecule.

The term "knowledge representation" refers to computational methods and data structures for encoding and storing real world knowledge, which may include a set of objects and the relationships between them, for example. Relationships are often defined by rules.

A "memory indexing structure" is a theoretical concept describing how the human brain may store memories and arrange them in order to facilitate subsequent retrieval.

The term "mass spectrometry" refers to a set of techniques for measuring the mass and charge of materials such as protein fragments, for example, such as by gathering data on trajectories of the materials/fragments through a measurement chamber. Mass spectrometry is particularly useful for measuring the composition (and/or relative abundance) of proteins and peptides in a sample.

A "microarray" or "DNA microarray" is a high-throughput hybridization technology that allows biologists to probe the activities of thousands of genes under diverse experimental conditions. Microarrays function by selective binding (hybridization) of probe DNA sequences on a microarray chip to fluorescently-tagged messenger RNA fragments from a biological sample. The amount of fluorescence detected at a probe position can be an indicator of the relative expression of the gene bound by that probe.

A "model", as used herein, refers to a data structure that contains all items, collections, and textual and graphical elements in a biological story; the computer representation of all data in Results Managers, Collection Manager, Story Editor, and Diagram Editor.

A "mouse sprite" refers to a displayed pointer on a computer screen, which corresponds to the movement of a mouse input to a graphical user interface.

A "naming convention" is a mutually agreed upon set of rules for naming of fields in experimental data sets.

A "narrative structure" refers to the underlying structure of a biological story, i.e. its partitioning of information into Theme, Player, and Explanation components; also the way in which many cognitive psychologists believe the human brain represents stories.

The term "Object Editor" refers to a software component for presenting, manipulating, and annotating the properties of items, collections, story nodes, and diagram nodes.

An "oncogene" refers to an altered gene that can lead to cancer.

An "oppose node" refers to an element in the Story Editor that can be used to document information and/or citations that dispute a claim made in a particular story node.

An "outline processor" is a software tool for textually building up an outline of a document, for example, the Outline View in Microsoft Word.

A "pathway" refers to a sequence of processes or mechanisms, such as biological processes or mechanisms that relay information between and within cells and/or produce biological products via biochemical reactions.

The term "Pathway Diagram" refers to a diagrammatic representation of a pathway, e.g., a biological pathway.

The term "peptide bond" refers to a polar covalent chemical bond joining two amino acids. Peptide bonds form the protein backbone.

"Persistent storage" refers to a computer medium for storing and retrieving data. Persistent storage typically can facilitated in a file or database.

A "Player" refers to an entity that plays an active role in a story; in the biological realm, a player is a biological entity that plays an active role in a biological story, e.g. a gene or protein that participates in a signal transduction pathway.

A "polymer" is a large molecule formed by linking together of smaller similar sub-units or "mers".

A "probe" (in a DNA microarray) refers to a DNA sequence that selectively binds (hybridizes) to particular DNA sequences in a biological sample, thus providing a measure of the relative expression level of a gene sequence of interest.

The term "promote" refers to an increase of the effects of a biological agent or a biological process.

A "protein" is a large polymer having one or more sequences of amino acid subunits joined by peptide bonds.

The term "protein abundance" refers to a measure of the amount of protein in a sample; often done as a relative abundance measure vs. a reference sample.

"Protein/DNA interaction" refers to a biological process wherein a protein regulates the expression of a gene, commonly by binding to promoter or inhibitor regions.

"Protein/Protein interaction" refers to a biological process whereby two or more proteins bind together and form complexes.

"Publish to Web" refers to a system facility for generating an interlinked set of HTML pages, where each item, each collection, and each element of a collection has its own Web page. This facility is useful for sharing a model with colleagues who are not using the present software system, since only a Web browser is required for viewing and navigating the information published.

A "Results Manager" refers to a software component and user interface for viewing and manipulating items.

A "sequence" refers to an ordered set of amino acids forming the backbone of a protein or of the nucleic acids forming the backbone of a gene.

The term "semantic overlay" or "data overlay" refers to a user interface technique for superimposing data from one view upon data in a different view; for example, overlaying gene expression ratios on top of diagram nodes in the Diagram Editor. This technique is useful for informally validating high-level explanations and hypotheses against detailed experimental data.

The term "signal transduction" refers to the relay of information from receptors in the cell membrane to the cell's response mechanism; the process by which stimulus energy is transformed into a response.

A "spreadsheet" is an outsize ledger sheet simulated electronically by a computer software application; used frequently to represent tabular data structures.

The term "Story Editor" refers to a software component for presenting and manipulating elements of a biological story, such as Players, Alternatives, and Explanations. The Story Editor can be thought of as a textual mechanism for putting together a biological story

A "story grammar" refers to a set of formal rules for organizing and interrelating the elements of a biological story; derived from research in cognitive psychology into story grammars as a way of structuring information in stories; related to forming memory indexing structures.

A "story node" refers to an element in the Story Editor, e.g. a Theme, Player, Interaction, Alternative, etc.

The term "story structure" refers to the manner in which elements of a biological story are organized and interrelated.

A "support node" when used in the context of the Story Editor is an element that can be used to document information and/or citations that support/reinforce a claim made in a particular story node.

The term "synthesis" refers to the combining of elements into a single or unified entity.

The term "syntax-directed editor" refers to a software tool for editing a document wherein the information added is constrained by grammatical rules. A syntax-directed editor is useful in helping a user structure a document for subsequent ease of reuse of the information. An example of a syntax-directed editor is the Story Editor.

The term "text mining" refers to a computational process of extracting higher-level knowledge from patterns of text in a document.

A "Theme" refers to a brief description of the overall gist of a biological story, such as might appear in the abstract of a journal article.

A "time course" refers to a series of measurements of a biological phenomenon taken over defined intervals of time, e.g. measurements of gene expression levels over 1, 3, 24, 48 hours in response to a treatment of a cell sample, such as exposure to ultraviolet light.

The term "time stamp" refers to a data field that represents the date and time that an annotation was made or a citation added to the system. A time stamp is stored by the system whenever an annotation is made or a citation is added, and is useful in tracking changes made by members of a work group.

The term "top-down hypothesis formulation" refers to the deductive process of deriving a high-level explanation or hypothesis, beginning with a mental model of a process and utilizing concepts and patterns inferred by "bottom up" data analysis.

The term "tools menu" refers to a user-interface mechanism for choosing one of a number of possible auxiliary operations, e.g. Publish to Web.

A "tree" is a hierarchical data structure and visualization in which nested levels of information are represented as branches and leaves of a tree. The Collection Manager and Story Editor both represent their data as trees.

The term "up-regulation", when used to describe gene expression, refers to an increase in the amount of messenger RNA (mRNA) formed by expression of a gene, with respect to a control.

The term "UniGene" refers to an experimental database system which automatically partitions DNA sequences into a non-redundant sets of gene-oriented clusters. Each UniGene cluster contains sequences that represent a unique gene, as well as related information such as the tissue types in which the gene has been expressed and chromosome location.

The term "URL" or "Uniform Resource Locator" refers to a protocol for specifying addresses on the Internet, used for locating resources such as Web pages.

A "Web page" refers to a single hypertext document, typically resident on the World Wide Web, that can incorporate text, graphics, sound, etc.

The "World Wide Web" is a system of extensively interlinked hypertext documents; a branch of the Internet.

The term "view" refers to a graphical presentation of a single visual perspective on a data set, for example a spreadsheet or tree diagram.

The term "visualization" or "information visualization" refers to an approach to exploratory data analysis that employs a variety of techniques which utilize human perception; techniques include graphical presentation of large amounts of data and facilities for interactively manipulating and exploring the data.The term "XML" or "Extended Markup Language" refers to a World Wide Web standard, derived from HTML, for representing structured information in hypertext documents. XML extends HTML in that documents are represented as rich tree structures; typically used for storing and transmitting data, rather than textual documents, between computer systems.

Biomedical researchers are inundated by data which exists in a myriad of forms and from a myriad of sources. From this vast amount of data, the researchers are faced with an unenviable task of culling meaningful data from a vast amount of "noise" or data which is not pertinent to the task at hand. Put another way, researchers seek to find needles of causality in haystacks of correlation.

To make the data meaningful and useful, researchers endeavor to construct a working explanation or "story" of what a gene or protein or other entity does, and how it interacts in pathways with other genes or proteins and their products, or in other chemical reactions or dynamic processes. For example, a story might portray a cascading set of proposed causal relationships between gene expression states. A specific example of this is a "biological story" built up by a team of biomedical researchers studying the influence of an oncogene (cancer-related gene) on a rare form of cancer [cite Khan et al, PNAS]. The researchers have run a number of experiments, using DNA microarrays to probe the influence of the PAX3-FHKR oncogene on thousands of genes under diverse experimental conditions. They have identified a number of affected genes, such as Myogenin and MyoD, which in turn may be playing influential roles in the cancer process. The researchers believe a cascade of activation events, initiated by the PAX3-FHKR oncogene, results in a pediatric muscle cancer, known as alveolar rhabdomyosarcoma (ARMS). The experimental data indicates that PAX3-FHKR directly induces (activates) the genes Myogenin and MyoD, and, through the actions of these two genes, induces the gene My14, a gene that is known to be associated with muscle cell growth and differentiation. The perturbation on the effects of My14 results in a failure of the muscle cells to differentiate and end the cell cycle. The failure of the muscle cells to exit the cell cycle results in cells proliferating in an uncontrolled manner (i.e. cancer).

The present invention provides tools and methods for constructing a story through iterative and interactive processes which may include any combination or all of the following: gathering information; organizing information into concepts and categories; formulating and documenting tentative explanations and hypotheses; documenting explanations and hypotheses via textual notes and graphical sketches; sharing explanations and hypotheses with colleagues; and incorporating verification and feedback from colleagues into the story.

To support these processes, the system according to the present invention provides a coordinated set of interactive information organization and synthesis tools, built upon a simple conceptual model using a free-form database and a narrative structure, incorporating and building items, collections, and biological stories.

Narrative structure is used based on findings in cognitive psychology and knowledge representation literature that people use story structure as a way of organizing and remembering information and that story creation is a fundamental process for constructing memory indexing structures, see for example, Thorndyke, P.W., "Cognitive Structures in Comprehension and Memory of Narrative Discourses", *Cognitive Psychology, 9,* 1977, pp. 77-110; and Schank, R, "Tell Me a Story: Narrative and Intelligence", Northwestern University Press, 1990; both of which are incorporated herein in their entireties, by reference thereto. The present invention applies a story grammar as a framework for organizing and indexing biological stories.

The free-form database model enables the user to more easily build up and evolve the information structure that supports a biological story. The strength of a free-form database model is that the entry of data can precede the creation of database tables; the user can "add data now and categorize later". The free-form model is the central data structure of the software system; it encompasses all the information including experimental data, annotations, categorization, and textual and graphical explanations of biological processes. Models can be saved and restored and a group of users can work with multiple models.

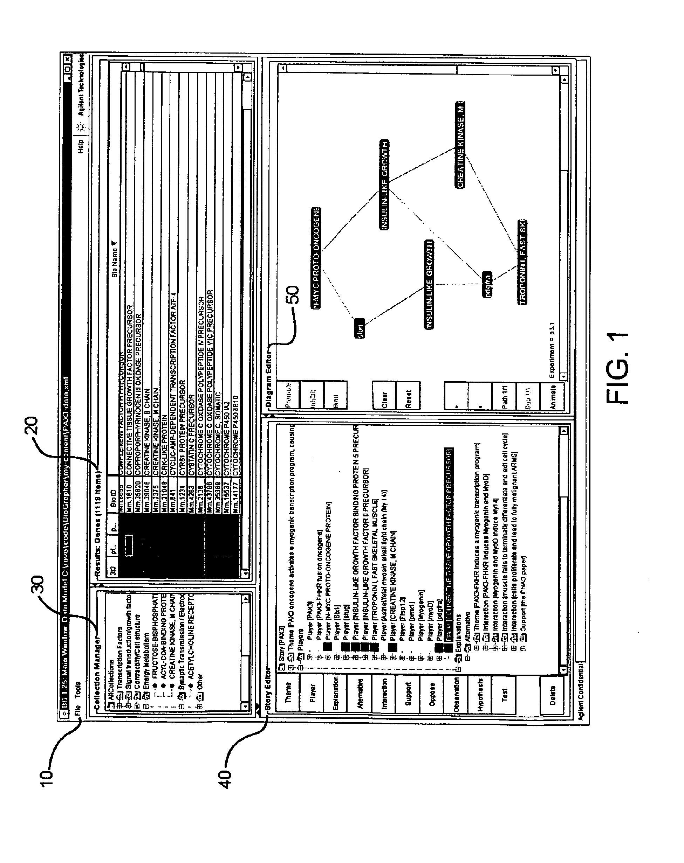

Fig. 1 shows examples of main windows of a system according to the present invention. The system may be built as a Java program to obtain portability across operating systems. Web and XML technology are used to represent and store information in a flexible fashion. While the implementation shown herein focuses on genes and gene expression, the techniques disclosed are equally useful for other biological data and problem areas, such as protein abundance, cell localization, protein/protein interactions, and protein/DNA interactions. Likewise, the techniques could be applied to other domains with problems concerning large numbers of interacting elements, e.g. the management of complex telecommunications networks.

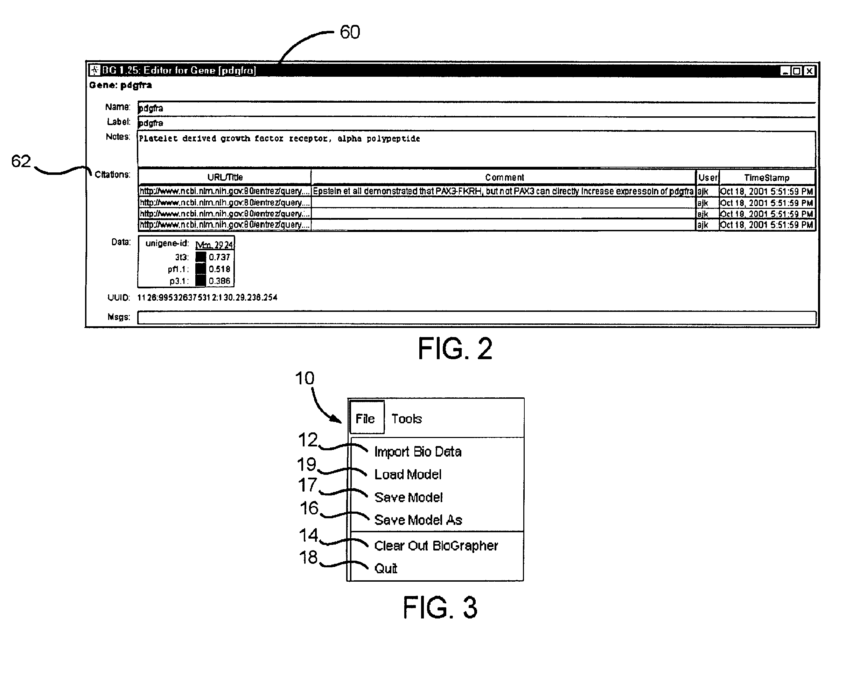

The main windows shown include: a Results Manager 20 for viewing detailed experimental results; a Collection Manager 30 for organizing experimental results and other information into groups and categories; a Story Editor 40, which provides a narrative structure for textually organizing information about the interrelationships and interactions amongst items and collections in biological processes, and a Diagram Editor 50, for graphically organizing information about the interrelationships and interactions amongst items and collections in biological processes. The Diagram Editor 50 also allows the construction of semantic overlays for validating high-level explanations against experimental results. An Object Editor 60 (Fig. 2) is provided for editing and annotating the properties and contents of items and collections.

Each window in Fig. 1 represents a different view into the overall model. These views and their associated data structures are closely and consistently coupled. An interactive change to an entity in any one view is reflected in all other views via a graphical user interface technique known as the Model/View/Controller paradigm, which is a specific type of event driven programming which may be carried out using the JAVA programming language, for example.

Model/View/Controller is a fundamental object-oriented programming paradigm which separates the actual data (represented by the view of the data) from the view of the data. The object (data structure) that represents the data has procedures that signal an event whenever the data is changed in any way, such as by deletion of data, addition of data, or modification of existing data, for example. By signaling an event, a message is sent indicating that the data has been changed.

The "Controller" aspect of the programming is implemented as a JAVA execution environment. A "listener" (a "listener" is a readily available JAVA construct) is defined and implemented by each view (e.g., results manager, collection manager, story editor, diagram editor, etc.) which registers with the controller to indicate that the viewer that is associated with each respective listener is interested in hearing about, or being notified when an event is signaled to indicate that data has been changed. The role of the controller is to coordinate the flow of events to listeners. When a listener receives a message (i.e., event) issued with regard to a change in data, it initiates procedures, which are specifically defined with respect to each viewer, as to what action to take when that particular message has been received. Thus, code that is specific to each viewer is executed substantially simultaneously to make changes to each view that represent the same change that was made to the data.

For example, a user may change the name of a collection in a collection manager. Assuming that this collection has already been added as a Player in the Story Editor prior to the user's change in the collection name, then a listener for the story editor receives the event that is generated when the collection name is changed. That listener then initiates execution of the procedures associated with the story editor which immediately make the collection name change in the story editor view. To the user, it appears that the collection name changes immediately, simultaneously with the change in the collection manager as the user manually makes the change in the collection manager.

Consistency and close coupling of multiple views enables the user to simultaneously view information from a variety of perspectives and across different levels of abstraction. This facilitates the discovery of unforeseen interrelationships, this aiding the process of piecing together explanations and hypotheses.

### ITEMS

Items are the basic "atomic" unit of information. They represent biological entities such as genes, proteins, sequences, and other gene products, or other entities in the case of a non-biological application of the system, such as network nodes or probes, for example. Items may contain detailed information about a biological entity, such as the quantitative results from an experimental assay. The user can create items by importing an experimental data set into the system. The user can import an experimental data set into a Results Manager 20 via the Import Bio Data item 12 on the File Menu 10 (see Fig. 3). Selecting the Import Bio Data menu item 12 results in a prompt for a file to import, via a "file chooser" dialog, which is similar in operation to the file chooser dialog in Microsoft Windows Explorer. The Import Bio Data operation imports a set of experimental data, such as gene expression data. Data is imported in the form of a spreadsheet with tab-separated columns. Each row of the spreadsheet data is read and used to create a new item that is added to the Results Manager 20. Properties and values are assigned to each item based upon the information imported from the appropriate columns.

In order to correctly make assignments to items and their data values, the program relies upon auxiliary file header information and conventions on how columns are named. The naming conventions in the current invention are specified in succeeding paragraphs. While the current invention supports naming conventions for gene expression data from microarray experiments, the import mechanism is generalized in principle and naming conventions can be defined to support import from other data sources, such as mass spectrometry data, or telecommunications data, for example.

The imported data files must have two additional "header" lines pre-pended to the actual data:

# gene data version 1.1

# unigene-id<tab>gene-name<tab><format><col>-<name><tab>...

Where <format> is one of:
- double--specifies that this column represents a Double value. This value will not be considered an experimental result (will not show up as a colored cell in the Results Manager 20 that encodes an experimental result, nor will it be used in any semantic overlays).
- int--specifies that this column represents an Integer value. This value will not be considered an experimental result (will not show up as a colored cell in the Results Manager 20 that encodes an experimental result, nor will it be used in any semantic overlays).
- text--specifies that this column represents a text value. All text up to the next \t (tab) or end of line is read and considered part of the text value. This value will not be considered an experimental result (will not show up as a colored cell in the Results Manager 20 that encodes an experimental result, nor will it be used in any semantic overlays).
- data-- specifies that this column represents a Double value. This value will be considered an experimental result and will be shown as a colored cell in Results Manager 20 and also used for color encodings in overlays.

<col> specifies the column where this data should be initially presented in the Results Manager 20,
<name> specifies the actual name of the column.

'unigene-id' is the header for the field that specifies the identifier in the Unigene database for the item
and 'gene name' is the header for the field that specifies the name of the item. For example,

# unigene-id gene-name data-1-UACC75 data-2-UACC89

Mismatched double quotes, single quotes, and extra ending white space are removed from names.

In the present invention, the software fills in, for each imported item with a Unigene-id field, a URL for the Unigene entry for that item, which can be traversed from within the Object Editor 60 for that item.

When a new data set is imported, the default operation is to add the new data to any existing data, so this may result in a duplication of items. The existing data set may be cleared by selecting the File => Clear out BioGrapher menu item 14.

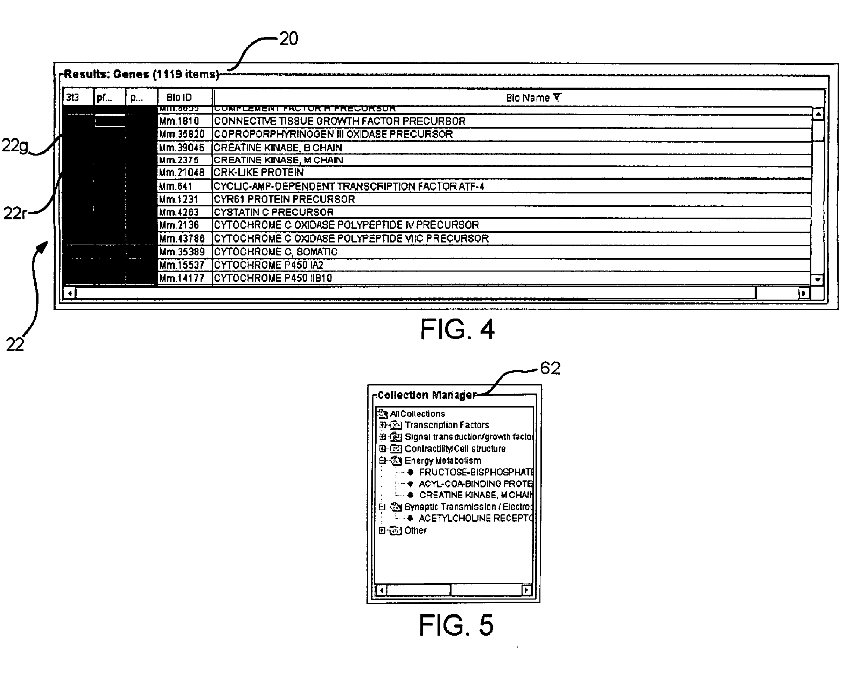

The upper-right pane in Fig. 1 contains a Results Manager 20 having a viewer (Results:Genes) for a data set of items. The Results Manager 20 is also shown in Fig. 4. In the example in Fig. 1, the data is drawn from several DNA microarray experiments. However, the data can be imported from a variety of experimental sources, for example relative protein abundance measures derived from mass spectrometry. Also, there can be multiple Results Manager 20 panes resident in the system at any time.

In the Results Manager 20 in Fig. 4, each row represents an individual item, such as a gene or protein. Each column represents an attribute of the item. An attribute of an item can be a property, such as its name, or an experimental condition, e.g. a therapeutic treatment or a tissue sample. Each cell in the Results Manager 20 (i.e. each row/column intersection) represents a value for that attribute of the item. In the leftmost columns in the Results Manager 20 of Figure 4, that value is a gene expression ratio. This ratio is a measure of the degree to which a gene is differentially expressed (or "turned on") in an experimental sample (versus a reference sample). For example, one might use DNA microarrays to measure expression levels of many thousands of genes across a set of different tumor tissues, contrasting each with gene expression levels for normal tissue. Many bioinformatics tools and databases store gene expression data in this form, so it is relatively straightforward to import gene expression data into the software. In this example, expression ratios 22 are represented by a color encoding which runs from green 22g (highly down-regulated) to red 22r (highly up-regulated). The Results Manager 20 may be sorted, using the values of any column as the sort key (not shown), by clicking on the column heading. The sort key is an internal construct used by the software, rather than an entity displayed in the user interface.

Items also serve as repositories for links to public data, such as literature citations. The user can move Web-based information for a gene into the item representing that gene by dragging and dropping (or copying and pasting) text and URLs from a Web page (e.g., an NCBI Genbank entry for a gene) onto the appropriate item. In addition to providing ways for the user to manually enter links to items, the system can also semi-automatically populate items with links to detailed data. For example, knowledge discovery and data mining tools can be utilized to retrieve pertinent literature references and database entries for an item. Further examples of knowledge discovery and data mining tools can be found in commonly owned, co-pending Application (Application Serial No., not yet assigned; Attorney's Docket No. 10020142-1) filed concurrently herewith and titled "Biotechnology Information Naming System", and in commonly owned, co-pending Application No. 10/033,823, filed December 19, 2001 and titled "Domain Specific Knowledge-Based Metasearch System and Methods of Using", both of which are incorporated herein, in their entireties, by reference thereto.

### COLLECTIONS

In order to build new abstractions, it is often useful for the user to group together chunks of related information. For example, a set of genes known to influence muscle cell differentiation may be thought of, manipulated, and annotated together as a single group or "concept". For example, proteins which all belong to the same family, e.g. growth factors, might for purposes of efficiency or convenience be thought of, manipulated, and annotated as a single group, rather than as individual proteins. The system supports these groupings through constructs known as collections. Collections are free-form sets of items. Collections are typically user-created, but can also be programmatically created, e.g. from the results of text mining.

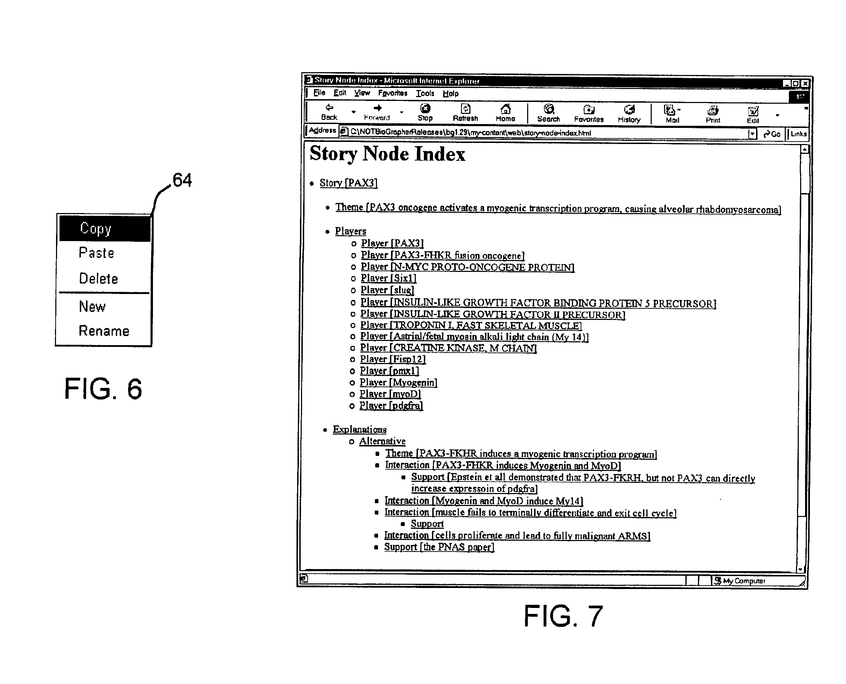

The user can group items into collections by dragging and dropping items from the Results Manager 20 onto the desired collection in the Collection Manager 60. Fig. 5 shows a Collection Manager window 62, which displays a tree view of collections; and functions in a way that is analogous to the tree view of folders in Windows Explorer. The user can create a new collection by pressing the right mouse button in the Collection Manager, then selecting the "New" item on the Collection Manager menu 64 shown in Fig. 6.

The Collection Manager 60 can also populate collections semi-automatically. One mechanism is by searching experimental data in the Results Manager 20 on a specified term or phrase. Using a dialogue box, the user enters a biological term of interest, for example, "kinase," and a collection will be built consisting of items in the Results Manager 20 whose names have a match for that term. Likewise, new collections can be formed by text mining of scientific literature, for example by looking for biological entities whose names co-occur frequently in journal articles. Commonly owned, co-pending Application (Application No. not yet assigned; Attorney's Docket No. 10020151-1) filed concurrently herewith and titled "System, Tools and Methods to Facilitate Identification and Organization of New Information Based on Context of User's Existing Information" provides tools for relevance ranking and filtering text that may be useful with the present invention, and is hereby incorporated, in its entirety, by reference thereto.

Collections are very malleable. Collections may be split or merged, items or groups of items may be added, deleted, or moved from one collection to another. Collections may be nested, i.e., a collection can contain other collections as well as items. Collections can be overlaid with detailed experimental data, for example by overlaying a set of expression levels on a collection of genes and highlighting the names of those genes whose expression levels exceed a certain threshold. Commonly owned, co-pending Application (Application No. not yet assigned; Attorney's Docket No. 10020167-1) filed concurrently herewith and titled "System and Methods for Extracting Pre-Existing Data From Multiple Formats and Representing Data in a Common Format for Making Overlays" provides tools and methods for performing overlays which may be useful with the present invention, and is hereby incorporated, in its entirety, by reference thereto.

As with items, collections can serve as repositories for links to detailed experimental data and public data, such as literature references. The advantage here over simply adding all the links to each of the members of the collection is that the link or annotation may be more relevant to the "concept" embodied by the collection, for example a link to information about the kinase family of proteins. The user moves Web-based information about a collection by dragging and dropping (or cutting and pasting) text and URLs from a Web page (e.g. an NCBI Genbank entry) onto the appropriate collection in the Collection Manager 60.

### BIOLOGICAL STORIES

Concurrently or consecutively with data import and annotation, the user can begin, with colleagues, to piece together higher-level explanations of biological processes by constructing biological stories, utilizing narrative structure to represent the state of the user's hypotheses and understandings. Narrative structure provides a framework for organizing information about the interrelationships and biological interactions amongst items and collections in biological processes. Biological stories can be used, for example, as templates for organizing and describing what is going on in the cell. A biological story can also be thought of as the representation of a hypothesis and the train of thought that produced that hypothesis.

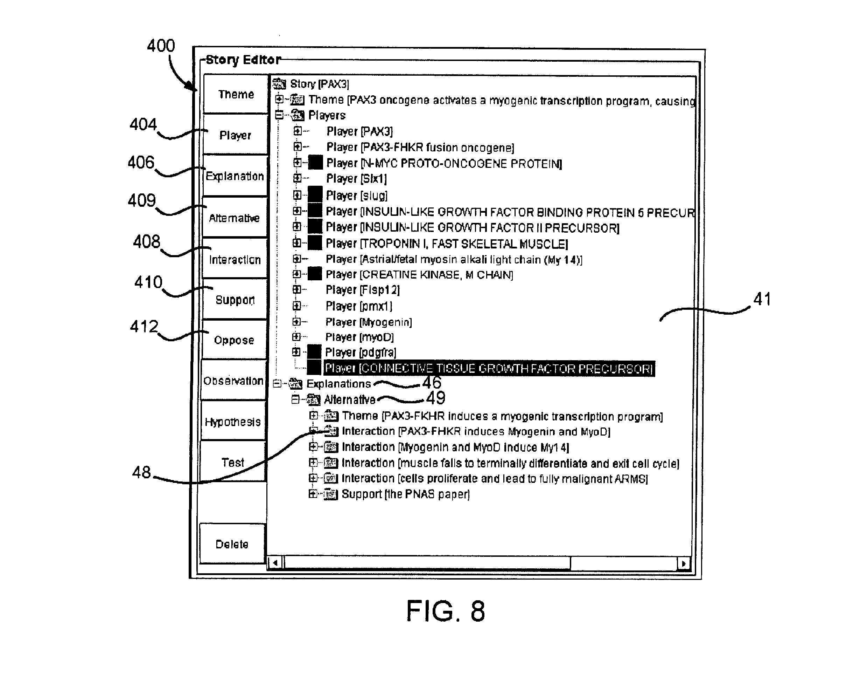

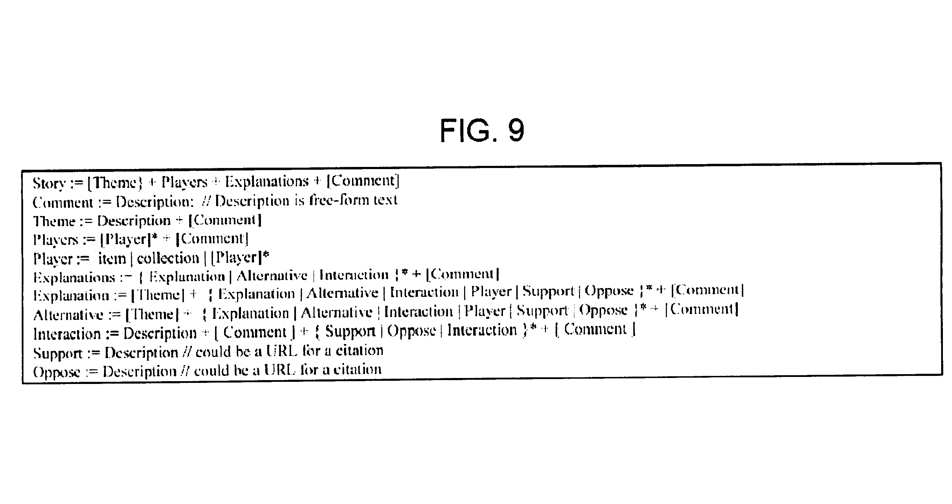

The user can piece together knowledge about a biological phenomenon and compose a biological story by using the Story Editor 40 component shown in Figs. 1 and 8. The Story Editor 40 is a syntax-directed tree editor, the syntax utilizing a story grammar, derived from cognitive psychology research and literary theory. The current invention provides a default story grammar; however, the grammar is user-configurable and the user(s) can substitute terms that are more intuitive or meaningful to them than those in the default story grammar. The default story grammar in the current invention is shown in Fig. 9.

A biological story includes three main sections: a Theme 42, a list of one or more Players 44, and a set of Explanations 46. The Theme 42 is a brief description of the overall gist of a biological story, such as might appear in the abstract of a journal article. The Players 44 comprise biological entities that play a role in the biological process being described in the story, for example genes and proteins, or collections of genes and/or proteins. Explanations 46 describe the "plot" of the story; they are essentially a set of evolving hypotheses about what processes may be occurring in a living cell, which are implied by the experimental data associated with the story.

An Explanation 46 can include one or more Interactions 48, basically steps in the process that is being described; for example, "PAX3-FKHR induces MY14". Different hypotheses can be represented by Alternatives 49, which specify different sets of possible Interactions 48. This is often useful in formative stages of an investigation, where there may be several plausible explanations for a particular biological phenomenon.

The user can document the reasoning behind Theme 42, Explanation 46, Interaction 48, and/or Alternative 49 story "elements", also referred to in this document as story "nodes", via Support and Oppose story elements. For example, the biologist can use a Support node to provide a citation from the literature that provides supportive evidence for the claims made in the Alternative 49. Likewise, the biologist can use an Oppose story node to provide a citation from the literature that provides evidence that disputes a claim.

The Story Editor 40 is a syntax-directed editor in which a biological story is represented by a tree structure. In this way, it is like an "outline processor". The tree appears on a canvas 41 on the right side of the Story Editor 40. Descriptions of biological phenomena are added to this tree, with nodes that correspond to the elements of narrative structure, i.e. Players 44, Explanations 46, etc. On the left side of the Story Editor is a set of buttons 400, which are used for adding nodes to (or deleting nodes from) the tree. Story nodes can be added to and deleted from the tree and textual descriptions can be added to story nodes in the tree. Textual descriptions can be added to any node by either editing the node's label in place or by invoking an Object Editor 60 interface, described in detail in a later section. Each story node represents an element of narrative structure: for example, a Player 44, Explanation 46 or Interaction 48.

A story node can be added by pressing a button in the Story Editor 40, for example pressing the Player button 404 to add a Player. For any story node in the story, there is a valid set of story nodes that can be nested below it. For example, it is valid to add a Player 44 to the Players node, but not to the Theme node. When a story node is added, the buttons representing the valid story nodes that can be nested below it are enabled, whereas the non-valid story nodes are disabled (grayed out).

The user typically starts building up a biological story by specifying the Players 44 in the story. Alternatively, an existing story may be imported into the present system and displayed in the Story Editor 40. The Players 44 in a biological story can be either items or collections. Players 44 may be added to a story by dragging and dropping (or cutting/copying and pasting) them from the Results Manager 20 and/or the Collection Manager 30, for example, when a story is being built or modified. Players 44 can also be added by pressing the Player button 404 and then adding descriptive text to the added element, as described above.

In its simplest form, the "plot" of a biological story represents a sequence or set of Explanations 46, which in turn contain a sequence or set of Interactions 48. The user creates Explanations 46 by selecting the Explanation button 406 in the Story Editor 40, which causes an Explanation node to be added to the biological story. The user then enters a textual description of the biological Explanation 46 by either editing the node's label in place or by invoking an Object Editor 60 interface that provides for detailed annotation of any node.

The user creates Interactions 48 by selecting the Interaction button 408 in the Story Editor 40, which causes an Interaction node to be added to the biological story. The user then enters a textual description of the biological Interaction 48 by either editing the node's label in place or by invoking an Object Editor 60 interface that provides for detailed annotation of any node.

In a situation where there may be more than one possible explanation for a sequence of events, alternative hypotheses for what is going on may be generated and tracked. This is often the case in the early phases of investigation, where there often are several possible explanations for a phenomenon. The user can add and keep track of all of the alternative hypotheses, and evolve them as the understanding of events becomes refined. To represent an alternative hypothesis, an Alternative node is added to the Explanations 46 of the biological story, or to a specific Explanation 46 or Interaction 48, by selecting the Alternative button 409. Then an alternative sequence of Explanations and/or Interactions can be added to that Alternative.

Since the user typically will have assumptions or evidence underlying different hypotheses, it is useful to keep track of these assumptions and evidence. The user can add a Support node to a Theme 42, Explanation 46, Player 44, Alternative 49, or Interaction 48 by selecting the Support button 410, and inputting that information under the appropriate node. Similarly, information that contradicts a hypothesis may be tracked. This is done by adding an Oppose node in the same manner as described above with regard to a Support node, except that the Oppose button 412 is selected to accomplish this task. Textual information may be added to the Support and/or Oppose node by either editing the node's label in place or by invoking an Object Editor 60 interface that provides for detailed annotation of any node. Database and literature citations may be added to the Support and/or Oppose nodes by dragging and dropping a URL from a Web page onto a Support or Oppose node, or onto the Object Editor 60 interface for that node.

### PUTTING THE STORY TOGETHER GRAPHICALLY

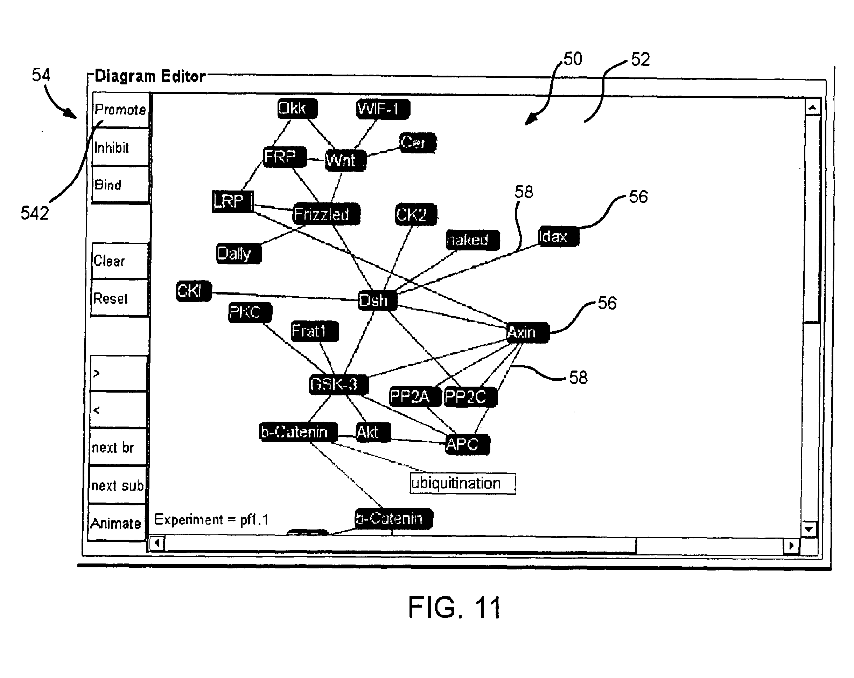

Using the Story Editor component 40, the user can build up a structured textual representation of a biological story. However, many people think graphically about stories and often use sketches and diagrams to represent their thinking about an explanation they are piecing together. This invention provides a Diagram Editor component 50, shown in Figs. 1 and 11, which may be used to put together a biological story pictorially. An analogy can be drawn here to Computer-Aided Circuit Design (CAD) software, particularly to CAD schematic capture tools, in that the biologist uses the Diagram Editor 50 to sketch out a representation of the "circuitry" of a biological process, such as might be found in a signal transduction pathway.

The Diagram Editor 50 is general and extensible and can be used to represent a variety of biological processes that can be expressed in diagrammatic form, for example biochemical pathways and/or protein/protein interaction maps. Likewise, the Diagram Editor 50 can be generalized to represent diagrams in other domains, such as telecommunications network diagrams.

The Diagram Editor component 50 includes a canvas 52 on the right and a set of buttons 54 on the left for adding elements. In the Diagram Editor component 50, the user can put together diagrams representing relationships between biological entities. These biological entities can correspond to items in the Results Manager 20, collections in the Collection Manager 30, Players 44 in the Story Editor 40, or any arbitrary information added to the Diagram Editor 50 by the user (or added programmatically). These biological entities and their relationships can be thought of as the "nouns" and "verbs" of the biological story. In the present invention, the "nouns" are represented by the biological entities and the "verbs" are represented by the interactions between them. In the Diagram Editor 50, the "nouns" are implemented as Diagram Nodes 56 and the "verbs" are implemented as Diagram Interactions 58.

The pictorial story can be built up by dragging and dropping items, collections, and/or Players 44 onto the Diagram Editor panel (canvas 52), or by adding an arbitrary diagram node 56 (either manually via a context-sensitive menu or programmatically via data/text mining software). When dragging and dropping onto the canvas, a graphical icon, representing the biological entity, appears at the drop point. There is a set of predefined "verbs" which are used to specify a relationship between "nouns", for example Inhibits, Promotes, or BindsTo. Commonly owned, co-pending Application (Application No. not yet assigned; Attorney's Docket No. 10020150-1) filed concurrently herewith and titled "System and Methods for Extracting Semantics from Images" provides tools and methods for extracting semantics from a static graphic image of a biological model and for converting the static image to an editable biological model which may be useful with the present invention, and is hereby incorporated, in its entirety, by reference thereto

Two "nouns" are connected with a "verb" by selecting the "verb" on the menu (e.g. by pressing a button labeled Promotes 542), then drawing a line between the two graphical icons representing the "nouns." Drawing is accomplished by positioning the mouse sprite over the first icon, pressing down on the mouse button, dragging the mouse sprite over to the second icon, then releasing the mouse button. A color-encoded arrow appears, connecting the two graphic icons, for example a red line represents the Promotes "verb." "Verbs" in the Diagram Editor 50 are directional; that is, a red arrow running from item A to item B indicates that "A Inhibits B," but not the converse.

There is a duality between graphical and textual storytelling. A textual story may be generated from the contents of the Diagram Editor component 50. In an analogous manner, diagram nodes 56 and diagram interactions 58 can be generated by parsing noun/verb phrases in the text of the story.

### SEMANTIC OVERLAYS

Often the user needs to do a "reality check" on a high-level story or explanation by comparing it with detailed experimental data. This is done to see if the experimental data is consistent with the claims made in the story. In other words, the "top-down" synthesis of the textual and/or graphical stories needs to be reconciled with the "bottom-up" exploration of the experimental data. One way of reconciling the synthesis with the data is to overlay items, collections, and biological stories with detailed experimental data. For example a set of expression levels may be overlaid on the Players 44 in a biological story and those genes whose expression levels exceed a certain threshold can be highlighted. In this way, the present invention provides a method for informally testing the hypotheses represented in biological stories. Such overlays are semantic, rather than literal, in that the meanings of the data, rather than their visual representations, are juxtaposed.

The present invention provides a method for constructing semantic overlays in the Diagram Editor component 50. If the items in the Results Manager 20 contain sets of quantitative values, for example expression levels from microarray experiments, then the biologist can "step through" each column of data and visualize the data values, such as expression levels, color-coded on top of the icons for those items in the Diagram Editor 50. Such "simulations" can be useful, for example, in inferring relationships between items, such as causal relationships inferred by "stepping through" time course data.

For example, in Fig. 1, many of the columns in the Results Manager 20 represent values from thousands of probes in DNA microarray experiments, where, for example, test samples may be compared with references samples (e.g., diseased tissue versus "normal" tissue) under various conditions. Cells (row/column intersections) in the Results Manager 20 that are colored reddish indicate an up-regulation of the gene, those that are colored greenish indicate a down-regulation of the gene, and a black color represents neutral, i.e., substantially no up or down regulation. Various shades and intensities of green and red result, which indicate the relative degree of up or down regulation of any particular probe. In the example, there were approximately 6000 rows in the matrix, although only a few have been shown in Fig. 1 for reasons of simplicity. Each column represents a different microarray experiment. This kind of color-encoding of expression values is often referred to as a "heat map".

In use, any column can be selected to overlay the values of that column onto the diagram in the Diagram Editor 50 and/or the Players 44 in the Story Editor 40. In the example shown in Fig. 1, when a column is selected, any genes having values in that column are matched up with their representations in the Diagram Editor 50 and the Story Editor 40. A visual representation of this overlay is displayed, wherein the overlaid data shows up in its representative color on each of the nodes in the Diagram Editor 50 as well as in the Story Editor 40. This holds true for each node in the pathway diagram that references an item in the experimental data, as well as each Player node in the Story Editor 40 that references an item in the experimental data.

A range of colors is mapped to a range of values in the data. Items that have similar values will have similar color schemes whereas items that are disparate will have different color schemes. The user can repeat this process, a column at a time from the values in the Results Manager 20, thereby stepping through all of the data resultant from the microarray experiments and analyzing each column in the same manner to verify correlating data and annotate discrepancies and outliers, by visualizing the expression levels, color-coded on top of the nodes for those items in the Diagram Editor 50 and/or Story Editer 40.

In addition to DNA microarray data, the present invention is capable of performing overlays of data from other diverse data sources, such as mass spectrometry or gel electrophoresis data. Moreover, this functionality can be generalized to other domains, for example in overlaying measurement data from telecommunications network probes onto network diagrams.

### ANNOTATION AND CITATIONS

To support users' keeping track of diverse pieces of information and to support team communication about the evolving information, this invention implements a rich annotation and citation facility. Every item, collection, story node, and diagram node or interaction can have arbitrary textual notes attached to it.

The present invention provides an Object Editor interface 60 for editing and annotating the properties and contents of biological entities or other items and collections. The Object Editor tool 60 is a form-based editor. By typing into fields in these forms, the user can add arbitrary annotations to the item or collection, as well as add annotations for each link to detailed information. For example, the user may want to add, as an annotation, a note that summarizes his/her current understanding of the function of a particular biological entity. The Object Editor 60 can be invoked by double-clicking on any biological object represented in the system. Fig. 2 shows the Object Editor 60 for an item.

Any and every item, collection, story node, and diagram node or interaction can have an arbitrary list of citations attached to it. The user can add citations by dragging/dropping URLs from a Web browser onto any object in the system or into the Citations field 62 of the Object Editor 60. Each citation can in turn have arbitrary textual notes attached to it. The user can add a note describing his or her reasoning or other context around their using a particular citation.

### SUPPORT FOR GROUP WORK

While the invention will be useful for an individual user in keeping track of information while building up explanations and hypotheses, some of its real power derives from the ability of the user to share biological stories with colleagues and collaborators. This is a way for the user to share the state of his/her thinking, receive feedback from colleagues, incorporate that feedback into the state of thinking, and, thus, refine the state of his/her thinking.

The present invention includes a number of facilities that support group work. Every annotation and citation is tagged with the name of the user who enters that annotation; it is also time-stamped. When the user adds an annotation to a citation, the annotation communicates to the group his or her reasoning behind using that citation. As described earlier, the support and oppose nodes in the Story Editor 40 enable users to record their lines of argumentation as alternative hypotheses are explored. It is very helpful to be able to articulate the lines of thought, and evidence related to those lines of thought, when working in groups.

The present invention further provides a repository of generated Web pages, described below, to support the sharing of biological stories and their supporting information.

### WEB REPOSITORY

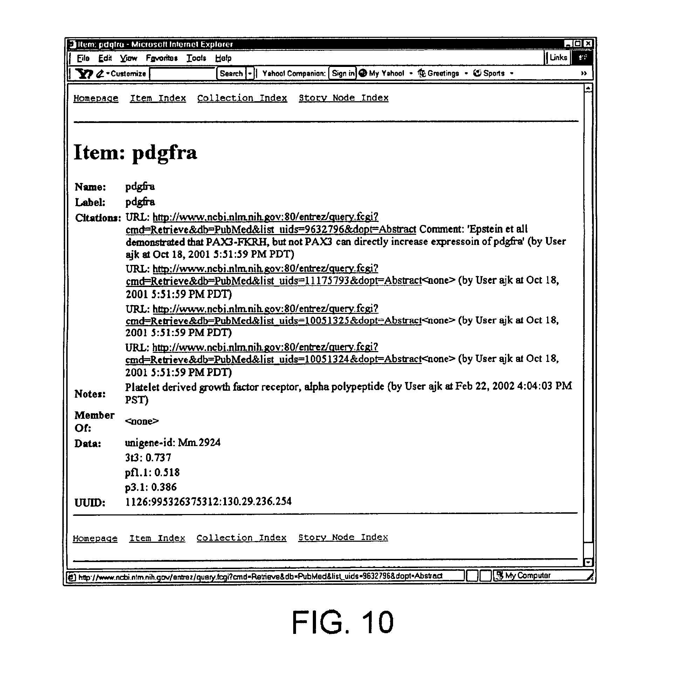

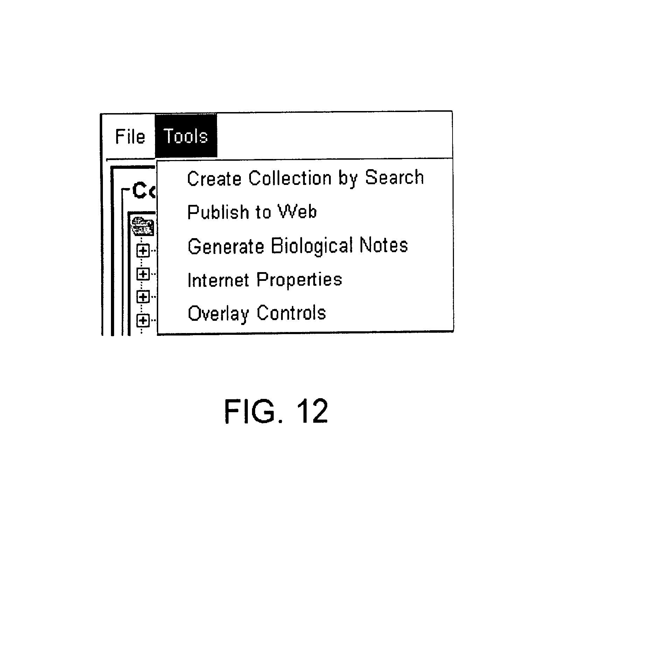

The present invention uses generated Web pages to represent the detailed information contained in its elements. The software generates an interlinked set of HTML pages, where each item, each collection, and each element of a story has its own Web page. A Web page for an item is shown in Fig. 10. When new information is associated with a data object, for example by dragging and dropping (or copying and pasting) a literature citation onto an item, that new information is incorporated into the Web page for that item. The user can navigate through this biological information space by selecting and following the links on the Web pages for items, collections, and stories. In addition to a specific Web page for each data object, there are index Web pages, one for the set of all items, one for the set of all collections, and one for the set of all story elements. The index page for the set of all story elements is shown in Fig. 7. A Web repository for a model can be created by selecting the "Publish To Web" menu item on the Tools menu, shown in Fig. 12.

To support the sharing of biological stories amongst groups of collaborating colleagues, the present invention generates a Web page for every node that appears in the Story Editor 40. Thus, every biological story can have its own Web page. The Players 44 displayed on the Web page for the biological story contain links to the Web pages for the items and collections represented by the Players 44 in the biological story. For example, the Web page in Fig. 10 points to the actual item for "pdgfra", not to the Player that references it. A player is actually a reference to an item, not the item itself. This distinction is important because the user can annotate a Player and item separately, which allows the use of annotations of the Player as a way to denote contextual information as it relates to the item's role in a particular story. That is, the same item could be a player in multiple stories (or even in multiple places, such as alternatives, in the same story). Therefore, having a distinct Player element allows the user to annotate specific information about the item's role in the story, distinct from direct annotations on the item itself. Thus, a collaborator that visits the Web page for a biological story can navigate throughout the entire context surrounding that biological story. The Web page is a richly interconnected map of the user's train of thinking in building up a particular set of explanations and/or hypotheses. Note that the collaborator does not specifically need to be using the software described in this invention in order to navigate through the Web repository for a story. Any Web browser will suffice for this purpose.

If a colleague is using the program described in this invention, rather than a Web browser, for navigating a biological story, then this colleague can serve as a "reviewer" and add annotations. This can done using the mechanisms for annotation described earlier. The software tags such annotations with the "reviewer's" name and also a time stamp, so that annotations from different colleagues can be distinguished and chronologically ordered.

### SAVING WORK IN PROGRESS

In the present invention, a model is the central data structure of the software system and it encompasses all the information including experimental data, annotations, categorization, and textual and graphical explanations of biological processes. Thus, a model embodies the current state of work-in-progress of the user. This state of work can be saved by invoking the "Save Model As" operation 16 on the File menu 10 shown in Fig. 3. All items, collections, and stories (both textual and graphical) are written to persistent storage, such as a file, using XML Web technology described at [http://w3.org]. All the links to detailed information associated with the items, collections, and stories are saved along with them. Other contextual information, such as the coordinates of nodes placed in the Diagram Editor 50 component, are also saved. All this information is restored the next time the program is run.

When saving a model, if there is not currently a persistent store (e.g. a file) for the model, then the user is prompted for a name for the model via a "file chooser" dialog. This is the case when the Save Model As operation 16 is invoked; the user will be prompted for a name for the model. In the case where the operation Save Model 17 has been invoked and there already exists a persistent store (e.g. a file) for that model, then the system will just overwrite the persistent store with the contents of the current model.

For safety purposes, the software will also prompt to save the current model upon exiting the program. Invoking the Quit item 18 on the File menu shown in Fig. 3 also causes the software to display a dialog box, asking to save changes.

The user can also load in an existing model from a persistent store (e.g. a file) by invoking the Load Model 19 operation on the File menu 10 shown in Fig. 3. Prior to loading in the model, the user will be prompted about whether to save changes made to the currently loaded model before loading in a model from persistent store. After that, the system will present a "file chooser" dialog, from which the user can choose an existing model to load.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, data type, network, user need, process, process step or steps, to the objective, spirit and scope of the present invention. All such modifications are intended to be within the scope of the claims appended hereto.

## Claims

1. A system for organizing information across external information objects, comprising:
a results manager (20) for importing and viewing detailed experimental results as one type of representation of external information objects;
a collection manager (30) for creating and manipulating collections of items representing external information objects;
a story editor (40) for providing a narrative structure for textually organizing information about interactions between items, collections or items and collections; and
a diagram editor (50) for incorporating items, collections or items and collections, into a graphical representation of a story;
wherein an update of information contained in any one of the components comprising the results manager, collection manager, story editor, and diagram editor is made in the remainder of the components.

2. The system of claim 1, wherein the update of information is automatically made in the remainder of the components.

3. The system of claim 1 or 2, further comprising
a controller means; and
at least one listener means associated with one of the results manager, the collection manager, the story editor and the diagram editor,
wherein the controller means detects changes in the information in one of the components and generates a message in response to the change of information, the message indicating which information was changed, and
wherein the listener means receives the message from the controller and causes the component with which the listener means is associated to update a specific information in the component on the basis of the information contained in the message.

4. The system of claim 3, wherein all components have associated a listener means.

5. The system of one of claims claim 1 to 3, further comprising:
means for overlaying information from one or more of the results manager (20), collection manager (30), story editor, and diagram editor (50) on one or more viewers of the results manager, collection manager, story editor, and diagram editor.

6. The system of claim 5, wherein in the results manager the experimental results are color encoded, and wherein the means for overlaying further comprises means for selecting a color encoding for an associated experimental result, and means for mapping the selected color encoding to elements in the story editor and/or in the diagram editor associated with the specific experimental result which color encoding was selected.

7. The system of one of claims 1 to 6, wherein the diagram editor (50) is adapted to automatically put together a diagram (52) representing a relationship between elements in the story editor (40).

8. The system of claim 7, wherein the diagram editor (50) comprises means for searching the story editor for nouns and verbs, wherein nouns represent specific elements in the story editor (40), and verbs represent specific interactions between the elements in the story editor (40), means for implementing the nouns as diagram nodes (56), and means for implementing verbs as diagram interactions (58).

9. The system of one of claims 1 to 8, further comprising means for importing the external information objects into the results manager (20), the external information objects being provided in first format, the external information objects being arranged in the results manager (20) in a second format, wherein said means for importing comprises means for converting the external information objects from the first format into the second format.

10. The system of claim 9, wherein the first format is a spreadsheet with tab-separated columns, and wherein the external information objects comprise an additional header, the header being in the form of:
unigene-id<tab>gene-name<tab><format><col>-<name><tab>
where
<format> is one of:
- double --specifies that this column represents a Double value,
- int --specifies that this column represents an Integer value,
- text --specifies that this column represents a text value,
- data -- specifies that this column represents a Double value,
<col> specifies the column where this data should be initially presented in the results manager,
<name> specifies the actual name of the column,
'unigene-id' is the header for the field that specifies the identifier in the Unigene database for the item.

11. The system of one of claims 1 to 10, wherein the collection manager comprises means for searching the results manager (20) for specific terms, and means for building the collection on the basis of matched terms in the results manager.

12. The system of claim 11, wherein the collection manager further comprises a dialog box for receiving the specific terms.

13. The system of one of claims 1 to 12, further comprising an object editor for adding as well as editing annotations to items, collections, stories, interactions, and graphical representations of stories.

14. The system of claim 13, further comprising means for tagging each said annotation with the user name who created it and with a time stamp indicating the time of creation of the annotation, respectively.

15. The system of one of claims 1 to 14, further comprising means for generating a web repository, the web repository including a web page for each item.

16. The system of one of claims 1 to 15, wherein the wherein the results manager, collection manager, story editor, and diagram editor support the display of the information.

17. A method of verifying and validating experimental data, the method comprising:
importing the experimental data into a results manager (20);
overlaying items selected from the results manager onto a textual story provided in a story editor (40) or onto a graphical story provided in a diagram editor (50);
comparing the overlaid items with the information in the textual story or the graphical story; and
indicating the result of the comparison in the story editor or in the diagram editor.

18. The method of claim 17, wherein the overlaying comprises selecting an item in the results manager or at least one node or interaction in the graphical story.

19. The method of claim 17 or 18, wherein in the results manager the experimental results are color encoded, and the overlaying further comprises selecting a color encoding for an associated experimental result, and mapping the selected color encoding to elements in the story editor and/or in the diagram editor associated with the specific experimental result which color encoding was selected.
